# FASCICULE DE BREVET EUROPEEN

(11) **EP 3 044 205 B1**
(45) Date de publication et mention de la délivrance du brevet: **27.12.2017**
(21) Numéro de dépôt: 14762013.2
(22) Date de dépôt: 11.09.2014
(51) Int. Cl.: C07C 313/04

(54) **PROCEDE DE PREPARATION DE DERIVES OXYSULFURES ET FLUORES PAR SULFINATION**
VERFAHREN ZUR HERSTELLUNG VON OXYSULFID UND FLUORIERTEN DERIVATEN DURCH SULFONIERUNG
METHOD FOR PREPARING OXYSULPHIDE AND FLUORINATED DERIVATIVES BY SULPHINATION

(30) Priorité: 12.09.2013 FR 1302126
(43) Date de publication de la demande: 20.07.2016
(73) Titulaire: Rhodia Operations, 75009 Paris (FR)
(72) Inventeur: REVELANT, Denis, 69740 Genas (FR); METZ, François, 69540 Irigny (FR); DAMBRIN, Valery, 69230 Saint Genis-laval (FR); CHAUVE, Marie, 69007 Lyon (FR)
(74) Mandataire: Menville, Laure
(86) Numéro de dépôt international: PCT/EP2014/069436
(87) Numéro de publication internationale: WO 2015/036504

(56) Documents cités:
- EP-A1- 0 735 023
- WO-A1-98/05609
- FR-A1- 2 900 403
- US-A1- 2009 137 840
- US-A1- 2012 022 269
- US-B2- 6 737 525
- ANDREAS KORNATH ET AL: "Vibrational Spectra and Structural Aspects of Fluorosulfites", INORGANIC CHEMISTRY, vol. 36, no. 24, 1 November 1997 (1997-11-01), pages 5570-5573, XP55372214, EASTON, US ISSN: 0020-1669, DOI: 10.1021/ic970315o

## Description

La présente invention a pour objet un procédé perfectionné de préparation de dérivés oxysulfurés et fluorés de formule EaSOOR par une réaction de sulfination.

Plus précisément, l'invention concerne la préparation d'acide fluorosulfinique et de ses sels, ainsi que d'acides fluoroalcanesulfiniques et sulfoniques et de leurs sels. L'invention vise particulièrement la préparation d'acides perfluoroalcanesulfiniques et sulfoniques, et préférentiellement des acides trifluorométhanesulfinique et trifluorométhanesulfonique sous forme salifiée.

Les acides perhalogénoalcanesulfoniques et plus particulièrement l'acide trifluorométhanesulfonique sont utilisés comme catalyseurs ou comme intermédiaires en synthèse organique.

Il est déjà connu de EP 0 735 023 et WO 2007/128893 (FR2900403) des procédés pour la synthèse de dérivés organiques oxysulfurés et fluorés, notamment de l'acide perfluorométhanesulfinique sous forme salifiée, par réaction dans un solvant organique polaire, d'un acide fluorocarboxylique au moins partiellement salifié et d'un oxyde de soufre.

Les procédés antérieurs nécessitent d'opérer avec une grande quantité d'oxyde de soufre. Or la concentration élevée en oxyde de soufre favorise les réactions compétitives et consécutives et par conséquent la production d'impuretés ce qui affecte sensiblement le rendement de la réaction. Aussi, les procédés antérieurs sont contraints d'opérer en limitant le taux de conversion pour éviter une dégradation trop importante du produit désiré.

Poursuivant ses recherches, la Demanderesse a trouvé que les procédés existants pouvaient encore être perfectionnés en contrôlant l'ajout d'oxyde de soufre dans le milieu réactionnel pour y maintenir la concentration en oxyde de soufre sensiblement constante tout en réduisant la quantité d'oxyde de soufre impliqué. La Demanderesse a ainsi mis au point, de manière surprenante, un nouveau procédé de production de dérivés oxysulfurés et fluorés de formule EaSOOR par une réaction de sulfination, lequel conduit à de nombreux avantages, en particulier, la mise au point d'un procédé à la fois plus respectueux de l'environnement en raison de l'emploi d'une quantité limitée et contrôlée d'oxyde de soufre, qui est un réactif toxique, et plus performant en raison d'une amélioration notable du taux de conversion, du rendement et de la productivité du procédé. Les réactions consécutives, défavorables, sont sensiblement réduites ce qui permet d'atteindre une sélectivité d'au moins 70% tout en convertissant quasi entièrement le produit de départ (taux de conversion atteignant au moins 90%), ce qui limite sensiblement les recyclages de produit de départ et simplifie considérablement l'aval du procédé.

La présente invention a pour objet un procédé de préparation d'un dérivé oxysulfuré et fluoré comprenant la mise en réaction, en présence d'un solvant organique :
i) d'au moins un composé de formule Ea-COOR (I), où Ea représente l'atome de fluor ou un groupe ayant de 1 à 10 atomes de carbone choisi parmi les fluoroalkyles, les perfluoroalkyles et les fluoroalkényles, et R représente l'hydrogène, un cation monovalent ou un groupe alkyle, et
ii) d'un oxyde de soufre,
   ledit procédé étant tel que le rapport molaire initial (oxyde de soufre/composé de formule (I)) est inférieur à 0,4 et la concentration en oxyde de soufre dissous dans le milieu réactionnel est maintenue constante durant toute la durée de la réaction à une valeur comprise entre 0,2% et 3% poids par un ajout continu dudit oxyde de soufre au milieu réactionnel.

Conformément à l'invention, on entend par « milieu réactionnel » le milieu dans lequel a lieu la réaction chimique de sulfination. Le milieu réactionnel comprend le solvant de la réaction et, en fonction de l'avancement de la réaction, les réactifs et / ou les produits de la réaction. Il peut comprendre en outre des additifs et des impuretés.

Conformément au procédé de l'invention, on fait réagir au moins un composé de formule Ea-COOR (I) avec un oxyde de soufre. Ledit composé de formule (I) peut notamment être un acide fluorocarboxylique (R = H dans la formule (I)), un sel dudit acide (R = cation monovalent dans la formule (I)) ou un ester dudit acide (R = groupe alkyle dans la formule (I) ayant de 1 à 10 atomes de carbone). Le procédé selon l'invention conduit à la préparation d'un dérivé oxysulfuré et fluoré de formule (II) Ea-SOOR, où R est défini comme ci-dessus. La réaction mise en oeuvre par le procédé selon l'invention est une réaction de sulfination.

De manière préférée, ledit composé de formule (I) est un sel d'un acide fluorocarboxylique dans lequel le groupe R est un cation monovalent choisi avantageusement dans le groupe constitué par les cations alcalins, les cations ammonium quaternaire et phosphonium quaternaire. De manière très préférée, il s'agit d'un cation alcalin, en particulier le sodium, le potassium, le césium et le rubidium, plus particulièrement le potassium. Comme cations ammonium ou phosphonium quaternaire, on met en oeuvre préférentiellement les tétraalkylammonium ou phosphonium, trialkylbenzylammonium ou phosphonium, les tétraarylammonium ou phosphonium, dont les groupes alkyles identiques ou différents représentent une chaîne alkyle linéaire ou ramifiée ayant de 4 à 12 atomes de carbone, de préférence de 4 à 6 atomes de carbone et le groupe aryle est avantageusement un groupe phényle. On choisit préférentiellement le cation tétrabutylphosphonium.

Conformément à l'invention, on entend par fluoroalkyle un groupe formé d'une chaîne hydrocarbonée linéaire ou ramifiée en C1-C10 comportant au moins un atome de fluor. On entend par perfluoroalkyle un groupe formé d'une chaîne linéaire ou ramifiée en C1-C10 comprenant seulement des atomes de fluor, en plus des atomes de carbone, et dépourvue d'atome d'hydrogène. On entend par fluoroalkényle un groupe formé d'une chaîne hydrocarbonée en C1-C10, linéaire ou ramifiée, comportant au moins un atome de fluor, et comprenant au moins une double liaison.

De manière préférée, le groupe Ea présent dans le composé de formule (I) est choisi parmi l'atome de fluor et un groupe ayant de 1 à 5 atomes de carbone choisi dans le groupe constitué par les fluoroalkyles, les perfluoroalkyles et les fluoroalkényles. De manière très préférée, le groupe Ea est choisi dans le groupe constitué par l'atome de fluor, le radical CH₂F-, le radical CHF₂- et le radical CF₃-. Il en résulte ainsi respectivement la préparation selon le procédé de l'invention de F-SO₂R, de CH₂F-SO₂R, de CHF₂-SO₂R et de CF₃-SO₂R, où R est défini comme ci-dessus, préférentiellement R est un cation alcalin, très préférentiellement le potassium. En particulier, le procédé selon la présente invention est un procédé de préparation de l'acide fluorométhanesulfinique sous forme salifiée, de l'acide difluorométhane-sulfinique sous forme salifiée, de l'acide trifluorométhanesulfinique sous forme salifiée.

Dans le procédé de l'invention, le composé de formule Ea-COOR (I) qu'on fait réagir peut être totalement ou en partie un composé recyclé, qui peut être obtenu par exemple par séparation à l'issue de la réaction du sulfination, ou qui peut provenir d'une étape ultérieure de synthèse, par exemple lors de la préparation d'un sel d'un composé sulfonimide, d'un composé fluoré ayant une fonction acide sulfonique -SO₂OH ou d'un composé fluoré ayant une fonction anhydride de l'acide sulfonique.

Le procédé selon l'invention est mis en oeuvre en présence d'oxyde de soufre, de préférence de dioxyde de soufre. L'oxyde de soufre est généralement mis en oeuvre sous forme gazeuse. Il peut être également introduit sous forme de solution, dans le solvant organique choisi pour la réaction, à une concentration variant généralement entre 1% et 10% poids, de préférence entre 3% et 6% poids.

Conformément au procédé de l'invention, le rapport molaire initial (oxyde de soufre/composé de formule (I)) est inférieur à 0,4, de préférence inférieur à 0,2. Une autre caractéristique essentielle du procédé de l'invention réside dans le maintien d'une concentration constante en oxyde de soufre dissous dans le milieu réactionnel durant toute la durée de la réaction, ladite concentration étant comprise entre 0,2% et 3% poids.
Par concentration constante, il convient d'entendre au sens de la présente invention, que ladite concentration peut varier de ± 20%, de préférence de ± 10%. Conformément au procédé de l'invention, le suivi de la concentration en oxyde de soufre dissous, préférentiellement en dioxyde de soufre dissous, dans le milieu réactionnel peut être assuré par une méthode analytique. L'analyse en ligne ou *in situ* du milieu réactionnel est un moyen qui permet de maintenir constante la concentration en oxyde de soufre dissous. Le maintien d'une concentration constante peut être mis en oeuvre par un ajout contrôlé et de manière continue d'oxyde de soufre au milieu réactionnel. L'ajout contrôlé d'oxyde de soufre au milieu réactionnel, ajusté selon les résultats fournis par l'analyse en ligne ou *in situ,* permet de manière avantageuse de convertir le composé de formule (I) en un composé oxysulfuré et fluoré tout en défavorisant sensiblement la chimie parasite liée à la dégradation du composé de formule (I) par l'oxyde de soufre, préférentiellement le dioxyde de soufre. Conformément au procédé de l'invention, toute méthode analytique permettant de mesurer en ligne (via boucle d'échantillonnage) ou *in situ* la concentration en oxyde de soufre dissous, préférentiellement en dioxyde de soufre dissous, dans le milieu réactionnel comprenant au moins un solvant organique tel que défini plus loin dans la présente description et un composé de formule (I) convient. De manière préférée, la concentration en oxyde de soufre dissous, préférentiellement en dioxyde de soufre dissous, dans le milieu réactionnel est suivie en ligne ou *in situ* par spectrométrie RAMAN, par spectroscopie proche infrarouge ou encore par spectroscopie UV. Par exemple, lorsque la concentration en oxyde de soufre dissous dans le milieu réactionnel est suivie par spectrométrie RAMAN, le réacteur est équipé d'une sonde Raman laquelle est reliée par une fibre optique au spectromètre Raman, ladite sonde permettant de suivre dans le milieu la concentration d'oxyde de soufre dissous.

Le procédé selon l'invention est mis en oeuvre en présence d'un solvant organique, préférentiellement aprotique et très avantageusement aprotique polaire. De manière préférée, ledit solvant comporte très peu d'impuretés porteuses d'hydrogène acide. On entend par solvant aprotique, un solvant qui, dans la théorie de Lewis n'a pas de protons à libérer. On fait appel, selon l'invention, à un solvant suffisamment stable dans les conditions réactionnelles. Il est souhaitable que le solvant solubilise le composé de formule (I) au moins partiellement, de préférence complètement. Ainsi, le solvant organique est choisi préférentiellement polaire. Il est ainsi préférable que le solvant aprotique polaire utilisable ait un moment dipolaire significatif. Ainsi, sa constante diélectrique relative ε est avantageusement au moins égale à environ 5. De préférence, sa constante diélectrique est inférieure ou égale à 50 et supérieure ou égale à 5, et est notamment comprise entre 30 et 40. Afin de déterminer si le solvant organique répond aux conditions de constante diélectrique énoncées ci-dessus, on peut se reporter, entre autres, aux tables de l'ouvrage : Techniques of Chemistry, II - Organic solvents - p. 536 et suivantes, 3^{ème} édition (1970).
On préfère en outre que les solvants utilisés dans le procédé de l'invention soient susceptibles de bien solvater les cations, ce qui signifie que le solvant présente certaines propriétés de basicité au sens de Lewis. Pour déterminer si un solvant satisfait à cette exigence, on apprécie sa basicité en se référant au "nombre donneur". On choisit un solvant organique polaire présentant un nombre donneur supérieur à 10, de préférence supérieur ou égal à 20. La borne supérieure ne présente aucun caractère critique. On choisit, de préférence, un solvant organique ayant un nombre donneur compris entre 10 et 30. On rappellera que le "nombre donneur" ou "donor number" désigné de manière abrégée DN, donne une indication sur le caractère nucléophile du solvant et révèle son aptitude à donner son doublet. Dans l'ouvrage de Christian REICHARDT, [Solvents and Solvent Effects in Organic Chemistry - VCH p.19 (1990)], on trouve la définition du "donor number" qui est défini comme le négatif (-ΔH) de l'enthalpie (Kcal/mol) de l'interaction entre le solvant et le pentachlorure d'antimoine, dans une solution diluée de dichloroéthane.
Selon la présente invention, le ou les solvants polaires ne présentent pas d'hydrogène acide, en particulier lorsque le caractère polaire du ou des solvants est obtenu par la présence de groupes électro-attracteurs, il est souhaitable qu'il n'y ait pas d'hydrogène sur l'atome en position α de la fonction électro-attractrice.
De manière plus générale, il est préférable que le pKa correspondant à la première acidité du solvant soit au moins égal à environ 20 ("environ" soulignant que seul le premier chiffre est significatif), avantageusement au moins égal à environ 25, de préférence compris entre 25 et 35.
Le caractère acide peut également être exprimé par l'indice accepteur AN du solvant, tel qu'il est défini par Christian REICHARDT, ["Solvents and solvent effects in Organic Chemistry", 2ème édition, VCH (RFA), 1990, pages 23-24]. Avantageusement, cet indice accepteur AN est inférieur à 20, en particulier inférieur à 18.
Les solvants répondant aux différents impératifs et donnant de bons résultats peuvent être notamment des solvants de type amide. Parmi les amides, on comprend aussi les amides à caractère particulier comme les urées tétrasubstituées et les lactames monosubstitués. Les amides sont de préférence substitués (disubstitués pour les amides ordinaires). On peut citer par exemple les amides tels que le N,N-diméthylformamide (DMF), le N,N-diéthylformamide ou encore le N,N-diméthylacétamide ; les dérivés de pyrrolidone, tels que la N-méthylpyrrolidone. Une autre catégorie particulièrement intéressante de solvant est constituée par les éthers, qu'ils soient symétriques ou non symétriques, qu'ils soient ouverts ou non. Dans la catégorie des éthers doivent être incorporés les différents dérivés des éthers de glycol tels que les différents glymes, le diglyme par exemple. Parmi les solvants précités, on met en oeuvre préférentiellement le DMF.
La quantité de solvant organique à mettre en oeuvre est déterminée en fonction de la nature du solvant organique choisi. Elle est déterminée de telle sorte que la concentration du composé de formule (I) dans le solvant organique soit de préférence comprise entre 1% et 40% en poids et plus préférentiellement entre 5% et 30%.
Selon des conditions préférées de mise en oeuvre du procédé de l'invention, il est souhaitable de contrôler la teneur en impuretés présentes dans le milieu réactionnel.
La teneur en atomes d'hydrogène labiles du système, ou plus exactement en protons libérables portés par ses divers composants, y compris leurs impuretés, doit être inférieure à la teneur en groupes fluorés libérés par la décomposition du composé de formule (I). Par atome d'hydrogène labile ou proton libérable, on entend un atome d'hydrogène qui est susceptible d'être arraché sous forme de proton par une base forte. En pratique, il s'agit des protons des fonctions acides qui présentent un pKa inférieur à environ 20. Plus la teneur en protons libérables sera faible, moins il y aura de risque de réaction parasite et meilleur sera le rendement. La teneur en protons libérables présents dans le milieu est au plus égale à 20% de la concentration molaire initiale dudit composé de formule (I). Avantageusement cette teneur est au plus égale à 10%, de préférence à 1% (en moles), par rapport à la teneur initiale en ledit composé de formule (I).

La principale molécule porteuse d'atomes d'hydrogène labiles est en général l'eau qui est susceptible de libérer jusqu'à deux protons par molécule. D'une manière générale il est préférable d'utiliser des réactifs et des solvants déshydratés, de manière que la teneur pondérale en eau de chacun des réactifs soit au plus égale à 1 pour 1000, par rapport à la masse totale dudit réactif. En fonction de l'ensemble des conditions réactionnelles, de telles teneurs en eau peuvent être satisfaisantes, mais dans certains cas, il peut être intéressant d'opérer à des niveaux plus bas, par exemple de l'ordre de 1 pour 10 000. Toutefois, il n'est pas nécessairement indispensable d'éliminer la totalité de l'eau et un rapport molaire eau/composé de formule (I) strictement inférieur à 10%, de préférence inférieur à 1% peut être toléré.
De plus, il est souhaitable que les impuretés métalliques soient en faibles quantités. Des éléments métalliques peuvent être présents en tant qu'impuretés apportées notamment par les réactifs, le solvant ou bien par les appareillages métalliques suite à une corrosion. Ainsi, afin de ne pas apporter de pollution métallique additionnelle, il importe en particulier que lorsque le composé de formule (I) est un sel d'acide fluorocarboxylique, celui-ci soit préparé par réaction d'une base avec l'acide fluorocarboxylique correspondant dans des conditions telles que la base soit introduite en une quantité voisine à ± 5% près, et de préférence égale à la quantité stoechiométrique. D'une manière plus générale, on peut indiquer que les deux catégories de métaux pouvant être essentiellement présents, à savoir les éléments de transition à deux états de valence (tels que le cuivre, le fer ou le chrome) et les éléments de la colonne VIII (notamment des métaux de la colonne du platine qui est le groupe constitué du platine, de l'osmium, de l'iridium, du palladium, du rhodium et du ruthénium), doivent être présents dans le milieu à une teneur exprimée par rapport à l'acide fluorocarboxylique au plus égale à 1000 ppm molaires, de préférence à 10 ppm molaires.

Conformément au procédé de l'invention, on met en contact le composé de formule (I), préférentiellement l'acide fluorocarboxylique sous forme salifiée, l'oxyde de soufre et le solvant organique. La mise en oeuvre peut être effectuée de manière semi-continue (ou semi-batch) ou d'une manière continue. De manière préférée, elle est effectuée de manière semi-continue.
Selon un mode de mise en oeuvre semi-continu, on peut introduire intégralement le composé de formule (I), préférentiellement le sel de l'acide fluorocarboxylique, dans le solvant organique puis ajouter en continu l'oxyde de soufre. L'oxyde de soufre peut être introduit sous forme gazeuse par absorption dans le milieu précité ou bien introduit également en solution dans un solvant organique, de préférence, celui de la réaction. De manière avantageuse, l'oxyde de soufre est ajouté après avoir préalablement chauffé la solution formée du solvant organique et du composé de formule (I), à une température comprise entre 50°C et 150°C.
Selon un mode de réalisation en continu, l'ensemble des réactifs est introduit en continu. On alimente généralement le dispositif où se produit la réaction par le composé de formule (I), préférentiellement le sel de l'acide fluorocarboxylique, en mélange avec le solvant organique et l'on introduit l'oxyde de soufre, lequel peut être ajouté dans la solution d'alimentation comprenant le composé de formule (I) et le solvant organique ou bien il peut être introduit à différents endroits de l'appareillage, l'arrivée pouvant être faite dans le ciel du réacteur ou dans la masse réactionnelle.

Qu'il soit opéré en mode semi-continu ou continu, le procédé selon l'invention comprend de préférence le contrôle en ligne ou *in situ* de la concentration en oxyde de soufre dissous durant toute la durée de la réaction de manière à maintenir constante ladite concentration, dans le milieu réactionnel, dans une gamme comprise entre 0,2% et 3% poids.

Le procédé selon l'invention est avantageusement opéré dans un appareillage permettant une mise en oeuvre en semi-continu ou en continu. En particulier, un réacteur parfaitement agité, une cascade de réacteurs parfaitement agités avantageusement équipés d'une double enveloppe ou un réacteur tubulaire équipé d'une double enveloppe dans lequel circule un fluide caloporteur dont les caractéristiques permettent d'atteindre la température réactionnelle souhaitée conviennent à la mise en oeuvre du procédé selon l'invention.

Selon un mode de réalisation préféré du procédé selon l'invention, de la silice est introduite dans le milieu réactionnel, préférentiellement dans une quantité telle qu'elle représente de 0,1% à 10% poids, de préférence de 0,5% à 10% poids dans le milieu réactionnel. De manière particulière, la silice est ajoutée dans la solution formée du solvant organique et du composé de formule (I) lorsque le procédé selon l'invention est mis en oeuvre en semi-continu. L'ajout de silice permet de diminuer sensiblement l'impact corrosif sur le réacteur des fluorures générés dans le milieu par la mise en oeuvre du procédé selon l'invention.

Conformément au procédé de l'invention, le chauffage du mélange réactionnel a lieu avantageusement à une température comprise entre 100°C et 200°C, de préférence entre 120°C et 160°C. La réaction de sulfination est avantageusement mise en oeuvre sous pression atmosphérique mais des pressions plus élevées peuvent être également utilisées. Ainsi, peut convenir une pression totale absolue choisie entre 1 et 20 bar et de préférence entre 1 et 3 bar.
Selon un autre mode de réalisation, la réaction peut être mise en oeuvre à une pression inférieure à la pression atmosphérique. La pression totale absolue peut être comprise entre 1 mbar et 999 mbar, de manière préférée entre 500 mbar et 950 mbar, et de manière plus préférée entre 800 mbar et 900 mbar.
La durée du chauffage peut varier largement en fonction de la température de réaction choisie. Elle peut varier entre environ 30 min à au plus une journée. Elle est avantageusement de plus d'une heure à moins de 20 heures et plus préférentiellement comprise entre 2 heures et 7 heures.
Selon le mode de réalisation en continu, le temps de séjour moyen qui est défini comme le rapport entre le volume de la masse réactionnelle et le débit d'alimentation, se situe entre 30 min et 10 heures, et plus préférentiellement entre 2 heures et 4 heures.
Lorsque ledit oxyde de soufre est du dioxyde de soufre, le mélange résultant de l'étape de sulfination peut comprendre deux phases : une phase liquide, où une partie au moins de l'acide Ea-COOH et du dioxyde de soufre sont dissous dans ledit solvant et une phase gazeuse contenant essentiellement du dioxyde de soufre et du gaz carbonique formé au cours de la réaction.
L'avancement de la réaction peut être contrôlé par le taux de conversion (TT) du composé de formule (I) qui est le rapport molaire de la quantité de composé de formule (I) disparu sur la quantité de composé de formule (I) initiale dans le milieu réactionnel, ce taux étant calculé aisément après dosage dudit composé de formule (I) restant dans le milieu.
Une fois atteint le taux de transformation désiré, le mélange réactionnel peut être traité de façon connue en soi pour séparer le produit obtenu, les produits de départ pouvant être recyclés pour produire une quantité supplémentaire du dérivé oxysulfuré et fluoré visé. Une ou plusieurs opérations de séparation liquide-solide peuvent être mises en oeuvre, par exemple pour séparer d'éventuelles impuretés solides du milieu réactionnel. Les techniques utilisées peuvent être la filtration sur différents types de supports, la centrifugation, la décantation et l'évaporation, cette liste n'étant pas exhaustive. Alternativement ou en plus, une ou des opérations de séparation liquide-liquide peuvent être mises en oeuvre pour séparer et/ou purifier le produit obtenu. Les techniques utilisées peuvent être la distillation, l'extraction liquide-liquide, la séparation par osmose inverse ou la séparation par des résines échangeuses d'ions, cette liste n'étant pas exhaustive. Ces opérations de séparation liquide-solide et liquide-liquide peuvent être conduites en régime continu ou discontinu, l'homme du métier pouvant choisir le régime le plus approprié.
Selon un mode réalisation, le procédé comprend en outre une étape postérieure à la réaction de sulfination consistant à séparer le composé de formule (I) n'ayant pas réagi et à recycler ce composé dans le procédé.

L'invention a également pour objet un procédé de préparation d'un composé choisi dans le groupe constitué par un composé sulfonimide (Ea-SO₂)₂NH, ses sels (Ea-SO₂)₂NMe (Me représentant un métal alcalin), un composé fluoré ayant une fonction acide sulfonique -SO₂OH et présentant une formule Ea-SO₂OH et un composé anhydride de formule (Ea-SO₂)₂O, Ea ayant la définition précisée plus haut dans la présente description, ledit procédé comprenant :
- une étape de préparation d'un dérivé oxysulfuré et fluoré de formule (II) selon le procédé décrit ci-avant,
- une étape dans laquelle ledit composé fluoré de formule (II) est utilisé en tant que composé réactif pour la synthèse d'un composé sulfonimide (Ea-SO₂)₂NH et de ses sels (Ea-SO₂)₂NMe (Me représentant un métal alcalin), d'un composé fluoré ayant une fonction acide sulfonique -SO₂OH et présentant une formule Ea-SO₂OH ou d'un composé anhydride de formule (Ea-SO₂)₂O, Ea ayant la définition précisée plus haut dans la présente description.

La présente invention a donc également pour objet un procédé de préparation d'un sel d'un composé sulfonimide de formule (Ea-SO₂)₂NMe à partir d'un dérivé oxysulfuré et fluoré de formule (II) comprenant :
a) la préparation d'un dérivé oxysulfuré et fluoré de formule (II) selon le procédé décrit ci-avant ;
b) une étape d'oxydation, par exemple par chloration, pour obtenir le composé (Ea-SO₂)X, où X est le chlore ou le fluor,
c) une étape d'ammonolyse de Ea-SO₂X en (Ea-SO₂)₂NH, NR"₃ ;
d) une étape d'acidification de (Ea-SO₂)₂NH, NR"₃ en (Ea-SO₂)₂NH ;
e) une étape de neutralisation, par une base de métal alcalin, de (Ea-SO₂)₂NH en (Ea-SO₂)₂NMe ; et
f) éventuellement une étape de séchage de (Ea-SO₂)₂NMe,
dans lequel Ea est défini comme ci-dessus, R" représente un groupe alkyle, linéaire ou branché, ayant de 1 à 20 atomes de carbone, Me représente un métal alcalin. De manière préférée, Me est le lithium.
Les étapes c), d), e) et f) sont connues de l'Homme du métier. En particulier, l'étape d'ammonolyse est décrite dans le brevet US 5.723.664. Les étapes d'oxydation, d'acidification, de neutralisation et de séchage sont des étapes classiques pouvant être effectuées dans les conditions connues de l'Homme du métier.

De façon spécifique, la présente invention peut aussi avoir pour objet l'enchainement des étapes (a) et (b) décrites ci-dessus. Ainsi, l'invention concerne un procédé de préparation d'un composé Ea-SO₂X, où X est le chlore ou le fluor, comprenant :
a) la préparation d'un dérivé oxysulfuré et fluoré de formule (II) selon le procédé décrit ci-avant ;
b) une étape d'oxydation, par exemple par chloration, pour obtenir le composé Ea-SO₂X, où X est le chlore ou le fluor.

De manière préférée, le dérivé oxysulfuré et fluoré de formule (II) est un sel de métal alcalin de trifluorométhylsulfinate de manière à pouvoir l'utiliser dans la synthèse du bis-(trifluorométhanesulfonyl)imide de formule (CF₃SO₂)₂NH et du bis-(trifluorométhanesulfonyl)imidure de lithium de formule (CF₃SO₂)₂NLi (LiTFSI).

De manière encore préférée, le dérivé oxysulfuré et fluoré de formule (II) présente la formule F-SO₂-R, où R est défini comme ci-dessus (R = H, cation monovalent ou groupe alkyle) de manière à pouvoir l'utiliser dans la synthèse du bis-(fluorosulfonyl)imide de formule (F-SO₂)₂NH et du bis-(fluorosulfonyl)imidure de lithium de formule (F-SO₂)₂NLi (LiFSI).

Les composés sulfonimides et leurs sels préparés selon les procédés décrits ci-dessus peuvent avantageusement être utilisés comme sels d'électrolyte, comme précurseurs d'agent antistatique ou encore comme précurseurs de tensio-actif. En particulier, lesdits composés peuvent avantageusement être employés comme électrolytes pour la fabrication de batteries, dans le domaine de l'électrochromisme, de l'électronique et de l'électrochimie. Ils sont avantageusement employés comme agents antistatiques pour la fabrication d'adhésifs sensibles à la pression (PSA : pressure sensitive adhesives). En tant qu'agent antistatique, ils peuvent encore être employés comme composants de lubrifiants. Ils sont utilisés dans les matériaux optiques tels que les appareils électroluminescents et entrent dans la composition de panneaux photovoltaïques. Ces utilisations sont également des objets de l'invention. En particulier, l'invention a pour objet un procédé de fabrication d'un dispositif électrochimique, de préférence une batterie, ledit procédé comprenant une étape de préparation d'un composé sulfonimide ou de ses sels selon le procédé décrit ci-avant, et une étape de fabrication du dispositif électrochimique dans lequel le composé sulfonimide ou ses sels est employé comme électrolyte.

Le dérivé oxysulfuré et fluoré de formule (II) préparé selon le procédé de l'invention est encore avantageusement utilisé pour la préparation, par oxydation, d'un composé fluoré de formule Ea-SO₂-OH où Ea est défini comme ci-dessus. A cet effet, on met par exemple ledit dérivé oxysulfuré et fluoré de formule (II) issu du procédé de l'invention en présence d'une solution aqueuse alcaline puis on procède à une étape d'acidification pour libérer le composé Ea-SO₂-OH, par exemple en utilisant une solution d'un acide minéral fort, tel que l'acide sulfurique ou l'acide chlorhydrique. La présente invention a donc également pour objet un procédé de préparation d'un composé fluoré de formule Ea-SO₂-OH à partir d'un dérivé oxysulfuré et fluoré de formule (II) comprenant :
a) la préparation d'un dérivé oxysulfuré et fluoré de formule (II) selon le procédé décrit ci-avant ;
b') l'oxydation du dérivé oxysulfuré et fluoré de formule (II) pour obtenir un composé fluoré de formule Ea-SO₂-OH, où Ea est défini comme ci-dessus.

De manière préférée, le dérivé oxysulfuré et fluoré de formule (II) est un sel de métal alcalin de trifluorométhylsulfinate (CF₃SO₂R où R est un métal alcalin) de manière à pouvoir l'utiliser dans la synthèse de l'acide trifluorométhanesulfonique (appelé acide triflique) de formule CF₃SO₂OH.

Le composé Ea-SO₂-OH ainsi obtenu est avantageusement transformé en anhydride de formule (Ea-SO₂)₂O. La réaction d'anhydrisation est connue de l'Homme du métier et est particulièrement décrite dans le brevet US 8.222.450. De manière préférée, le dérivé oxysulfuré et fluoré de formule (II) est un sel de métal alcalin de trifluorométhylsulfinate de manière à ce que l'anhydrisation de l'acide triflique conduise à la production de l'anhydride trifluorométhanesulfonique de formule (CF₃SO₂)₂O. La présente invention a également pour objet un procédé de préparation d'un composé anhydride de formule (Ea-SO₂)₂O, où Ea est défini comme ci-dessus, à partir d'un dérivé oxysulfuré et fluoré de formule (II) comprenant :
a) la préparation d'un dérivé oxysulfuré et fluoré de formule (II) selon le procédé décrit ci-avant ;
b') l'oxydation du dérivé oxysulfuré et fluoré de formule (II) pour obtenir un composé fluoré de formule Ea-SO₂-OH ;
c') l'anhydrisation du composé fluoré de formule Ea-SO₂-OH pour obtenir un composé anhydride de formule (Ea-SO₂)₂O.

Les composé fluoré de formule Ea-SO₂-OH et les composés anhydrides de formule (Ea-SO₂)₂O peuvent être utilisés dans diverses applications, notamment comme catalyseur acide, comme groupe de protection en synthèse organique, comme synthon dans les domaines de la pharmaceutique, l'agrochimie ou l'électronique, ou comme sel pour l'électronique.

Les exemples qui suivent, illustrent l'invention sans toutefois la limiter.
Le taux de conversion (TT) correspond au rapport entre le nombre de moles de substrat transformées et le nombre de moles de substrat engagées.
Le rendement (RR) correspond au rapport entre le nombre de moles de produit formées et le nombre de moles de substrat engagées.

### Exemple 1 (comparatif) : préparation de trifluorométhylsulfinate de potassium CF₃SO₂K par sulfination de trifluoroacétate de potassium en présence de SO₂ selon une mise en oeuvre en discontinu (batch)

Dans un réacteur de 500 cm³ équipé d'une double enveloppe, d'une agitation mécanique centrale et d'une sortie sur l'atmosphère et d'un condenseur acétone/carboglace permettant le reflux du dioxyde de soufre, on charge à température ambiante (environ 20°C), 125,5 g de diméthylformamide. On introduit 25,5 g de trifluoroacétate de potassium dans le DMF. On charge ensuite 6,9 g de dioxyde de soufre par l'intermédiaire d'un capillaire relié à une bouteille de dioxyde de soufre sous pression. On chauffe à 140°C sous pression atmosphérique. Le rapport molaire SO₂ / TFAK est égal à 0,64.

Au bout de 4 heures et 25 minutes, l'analyse chromatographique ionique donne les résultats suivants :
- Taux de conversion du trifluoroacétate de potassium : 57,1%
- Rendement en trifluorométhylsulfinate de potassium : 52,8%

### Exemple 2 (invention) : préparation de trifluorométhylsulfinate de potassium CF₃SO₂K par sulfination de trifluoroacétate de potassium en présence de SO₂ selon une mise en oeuvre en semi-continu.

Dans un réacteur de 500 ml double -enveloppé, équipé d'un condenseur à eau glycolée à -15°C, d'une agitation et de contre-pales on introduit à température ambiante :
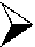 200 g de diméthylformamide (DMF) anhydre
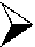 50 g de trifluoroacétate de potassium (TFAK) soit une concentration en TFAK égale à 20% poids dans le mélange DMF-TFAK.

Le réacteur est équipé d'une sonde RAMAN permettant de suivre dans le milieu la concentration de SO₂ dissous, cette sonde est reliée par fibre optique au spectromètre Raman.

Le milieu est agité et porté à une température de 100°C.

Par l'intermédiaire d'un tube plongeant relié à la bouteille de SO₂, on introduit en continu dans le réacteur, à travers une vanne micrométrique de réglage, une quantité de 1,25 g de SO₂ gazeux de manière à avoir une concentration en SO₂ dissous égale à 0,5% poids et un ratio molaire initial SO₂/TFAK de 0,059.

Tout en maintenant constante la concentration de SO₂ à 0,5% poids, on porte la température à 145°C. On laisse la réaction se dérouler pendant 5 heures en régulant la concentration de SO₂ à 0,5% poids.

Après 5 heures, le mélange réactionnel est refroidi et analysé par RMN et les résultats sont les suivants :
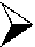 Taux de conversion du trifluoroacétate de potassium : 90%
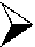 Rendement en trifluorométhylsulfinate de potassium : 64,8%

On observe une nette amélioration de la conversion et du rendement de réaction.

## Revendications

1. Procédé de préparation d'un dérivé oxysulfuré et fluoré de formule Ea-SOOR (II) comprenant la mise en réaction, en présence d'un solvant organique :
i) d'au moins un composé de formule Ea-COOR (I), où Ea représente l'atome de fluor ou un groupe ayant de 1 à 10 atomes de carbone choisi parmi les fluoroalkyles, les perfluoroalkyles et les fluoroalkényles et R représente l'hydrogène, un cation monovalent ou un groupe alkyle, et
ii) d'un oxyde de soufre,
ledit procédé étant tel que le rapport molaire initial (oxyde de soufre/composé de formule (I)) est inférieur à 0,4 et la concentration en oxyde de soufre dissous dans le milieu réactionnel est maintenue constante durant toute la durée de la réaction à une valeur comprise entre 0,2% et 3% poids par un ajout continu dudit oxyde de soufre au milieu réactionnel.

2. Procédé selon la revendication 1 dans lequel ledit composé de formule (I) est un sel d'un acide fluorocarboxylique dans lequel le groupe R est un cation monovalent choisi dans le groupe constitué par les cations alcalins, les cations ammonium quaternaire et phosphonium quaternaire, de préférence R est un cation alcalin.

3. Procédé selon la revendication 1 ou 2 dans lequel le groupe Ea est choisi dans le groupe constitué par l'atome de fluor, le radical CH₂F-, le radical CHF₂- et le radical CF₃-.

4. Procédé selon l'une des revendications 1 à 3 dans lequel l'oxyde de soufre est le dioxyde de soufre.

5. Procédé selon l'une des revendications 1 à 4 dans lequel le rapport molaire initial (oxyde de soufre/composé de formule (I)) est inférieur à 0,2.

6. Procédé selon l'une des revendications 1 à 5 dans lequel la concentration en oxyde de soufre dissous dans le milieu réactionnel est suivie en ligne ou *in situ* par spectrométrie RAMAN, par spectroscopie proche infrarouge ou encore par spectroscopie UV.

7. Procédé selon l'une des revendications 1 à 6 dans lequel ledit solvant organique est un solvant aprotique polaire, de préférence choisi parmi le N,N-diméthylformamide (DMF), le N,N-diéthylformamide ou encore le N,N-diméthylacétamide.

8. Procédé selon l'une des revendications 1 à 7 dans lequel la mise en oeuvre est effectuée de manière continue ou de manière semi-continue.

9. Procédé selon l'une des revendications 1 à 8 dans lequel la réaction est mise en oeuvre à une pression totale absolue choisie entre 1 et 20 bar, et de préférence entre 1 et 3 bar.

10. Procédé selon l'une des revendications 1 à 8 dans lequel la réaction est mise en oeuvre à une pression inférieure à la pression atmosphérique.

11. Procédé de préparation d'un composé choisi dans le groupe constitué par un composé sulfonimide (Ea-SO₂)₂NH, ses sels (Ea-SO₂)₂NMe (Me représentant un métal alcalin), un composé fluoré ayant une fonction acide sulfonique -SO₂OH et présentant une formule Ea-SO₂OH, et un composé anhydride de formule (Ea-SO₂)₂O, Ea représente l'atome de fluor ou un groupe ayant de 1 à 10 atomes de carbone choisi parmi les fluoroalkyles, les perfluoroalkyles et les fluoroalkényles, ledit procédé comprenant :
- une étape de préparation d'un dérivé oxysulfuré et fluoré de formule (II) selon le procédé selon l'une des revendications 1 à 10,
- une étape dans laquelle ledit composé fluoré de formule (II) est utilisé en tant que composé réactif pour la synthèse d'un composé sulfonimide (Ea-SO₂)₂NH et de ses sels (Ea-SO₂)₂NMe (Me représentant un métal alcalin), d'un composé fluoré ayant une fonction acide sulfonique -SO₂OH et présentant une formule Ea-SO₂OH, ou d'un composé anhydride de formule (Ea-SO₂)₂O.

12. Procédé selon la revendication 11, **caractérisé en ce qu'**il s'agit d'un procédé de préparation d'un sel d'un composé sulfonimide de formule (Ea-SO₂)₂NMe à partir d'un dérivé oxysulfuré et fluoré de formule (II) comprenant :
a) la préparation d'un dérivé oxysulfuré et fluoré de formule (II) selon le procédé selon l'une des revendications 1 à 10 ;
b) une étape d'oxydation, par exemple par chloration, pour obtenir le composé (Ea-SO₂)X, où X est le chlore ou le fluor,
c) une étape d'ammonolyse de Ea-SO₂X en (Ea-SO₂)₂NH, NR"₃ ;
d) une étape d'acidification de (Ea-SO₂)₂NH, NR"₃ en (Ea-SO₂)₂NH ;
e) une étape de neutralisation, par une base de métal alcalin, de (Ea-SO₂)₂NH en (Ea-SO₂)₂NMe ; et
f) éventuellement une étape de séchage de (Ea-SO₂)₂NMe,
R" représente un groupe alkyle, linéaire ou branché, ayant de 1 à 20 atomes de carbone, Me représente un métal alcalin.

13. Procédé selon la revendication 11, **caractérisé en ce qu'**il s'agit d'un procédé de préparation d'un composé fluoré de formule Ea-SO₂-OH à partir d'un dérivé oxysulfuré et fluoré de formule (II) comprenant :
a) la préparation d'un dérivé oxysulfuré et fluoré de formule (II) selon le procédé selon l'une des revendications 1 à 10 ;
b')-l'oxydation du dérivé oxysulfuré et fluoré de formule (II) pour obtenir un composé fluoré de formule Ea-SO₂-OH.

14. Procédé selon la revendication 11, **caractérisé en ce qu'**il s'agit d'un procédé de préparation d'un composé anhydride de formule (Ea-SO₂)₂O à partir d'un dérivé oxysulfuré et fluoré de formule (II) comprenant :
a) la préparation d'un dérivé oxysulfuré et fluoré de formule (II) selon le procédé selon l'une des revendications 1 à 10 ;
b') l'oxydation du dérivé oxysulfuré et fluoré de formule (II) pour obtenir un composé fluoré de formule Ea-SO₂-OH ;
c') l'anhydrisation du composé fluoré de formule Ea-SO₂-OH pour obtenir un composé anhydride de formule (Ea-SO₂)₂O.

## Patentansprüche

1. Verfahren zur Herstellung eines Fluoroxysulfid-Derivats der Formel Ea-SOOR (II), bei dem man in Gegenwart eines organischen Lösungsmittels:
i) mindestens eine Verbindung der Formel Ea-COOR (I), wobei Ea für das Fluoratom oder eine Gruppe mit 1 bis 10 Kohlenstoffatomen, die aus Fluoralkylgruppen, Perfluoralkylgruppen und Fluoralkenylgruppen ausgewählt ist, steht und R für Wasserstoff, ein einwertiges Kation oder eine Alkylgruppe steht, und
ii) ein Schwefeloxid
umsetzt, wobei das Verfahren so beschaffen ist, dass das anfängliche Molverhältnis (Schwefeloxid/Verbindung der Formel (I)) kleiner als 0,4 ist und die Konzentration von in dem Reaktionsmedium gelöstem Schwefeloxid während der gesamten Dauer der Umsetzung durch kontinuierliche Zugabe des Schwefeloxid zum Reaktionsmedium bei einem Wert zwischen 0,2 und 3 Gew.-% konstant gehalten wird.

2. Verfahren nach Anspruch 1, bei dem es sich bei der Verbindung der Formel (I) um ein Salz einer Fluorcarbonsäure handelt, in dem die Gruppe R ein einwertiges Kation aus der Gruppe bestehend aus Alkalikationen, quaternären Ammoniumkationen und quaternären Phosphoniumkationen ist und R vorzugsweise ein Alkalikation ist.

3. Verfahren nach Anspruch 1 oder 2, wobei die Gruppe Ea aus der Gruppe bestehend aus dem Fluoratom, dem CH₂F-Rest, dem CHF₂-Rest und dem CF₃-Rest ausgewählt ist.

4. Verfahren nach einem der Ansprüche 1 bis 3, bei dem es sich bei dem Schwefeloxid um Schwefeldioxid handelt.

5. Verfahren nach einem der Ansprüche 1 bis 4, bei dem das anfängliche Molverhältnis (Schwefeloxid/Verbindung der Formel (I)) kleiner als 0,2 ist.

6. Verfahren nach einem der Ansprüche 1 bis 5, bei dem die Konzentration von in dem Reaktionsmedium gelöstem Schwefeloxid in-line oder in situ durch Raman-Spektrometrie, Nahinfrarot-Spektroskopie oder UV-Spektroskopie verfolgt wird.

7. Verfahren nach einem der Ansprüche 1 bis 6, bei dem es sich bei dem organischen Lösungsmittel um ein polares aprotisches Lösungsmittel handelt, das vorzugsweise aus N,N-Dimethylformamid (DMF), N,N-Diethylformamid oder N,N-Dimethylacetamid ausgewählt wird.

8. Verfahren nach einem der Ansprüche 1 bis 7, dass kontinuierlich oder halbkontinuierlich durchgeführt wird.

9. Verfahren nach einem der Ansprüche 1 bis 8, bei dem die Umsetzung bei einem absoluten Gesamtdruck zwischen 1 und 20 bar und vorzugsweise zwischen 1 und 3 bar durchgeführt wird.

10. Verfahren nach einem der Ansprüche 1 bis 8, bei dem die Umsetzung bei einem unter Normaldruck liegenden Druck durchgeführt wird.

11. Verfahren zur Herstellung einer Verbindung aus der Gruppe bestehend aus einer Sulfonimidverbindung (Ea-SO₂)₂NH, deren Salzen (Ea-SO₂)₂NMe (wobei Me für ein Alkalimetall steht), einer Fluorverbindung mit einer Sulfonsäurefunktion -SO₂OH und der Formel Ea-SO₂OH und einer Anhydridverbindung der Formel (Ea-SO₂)₂O, wobei Ea für das Fluoratom oder eine Gruppe mit 1 bis 10 Kohlenstoffatomen, die aus Fluoralkylgruppen, Perfluoralkylgruppen und Fluoralkenylgruppen ausgewählt ist, steht, wobei das Verfahren Folgendes umfasst:
- einen Schritt der Herstellung eines Fluoroxysulfid-Derivats der Formel (II) gemäß dem Verfahren nach einem der Ansprüche 1 bis 10,
- einen Schritt, bei dem die Fluorverbindung der Formel (II) als reaktive Verbindung für die Synthese einer Sulfonimidverbindung (Ea-SO₂)₂NH und deren Salzen (Ea-SO₂)₂NMe (wobei Me für ein Alkalimetall steht), einer Fluorverbindung mit einer Sulfonsäurefunktion -SO₂OH und der Formel Ea-SO₂OH oder einer Anhydridverbindung der Formel (Ea-SO₂)₂O verwendet wird.

12. Verfahren nach Anspruch 11, **dadurch gekennzeichnet, dass** es sich um ein Verfahren zur Herstellung eines Salzes einer Sulfonimidverbindung der Formel (Ea-SO₂)₂NMe aus einem Fluoroxysulfid-Derivat der Formel (II) handelt, das Folgendes umfasst:
a) einen Schritt der Herstellung eines Fluoroxysulfid-Derivats der Formel (II) gemäß dem Verfahren nach einem der Ansprüche 1 bis 10;
b) einen Schritt der Oxidation, beispielsweise durch Chlorierung, zum Erhalt der Verbindung (Ea-SO₂)X, wobei X für Chlor oder Fluor steht;
c) einen Schritt der Ammonolyse von Ea-SO₂X zu (Ea-SO₂)₂NH, NR"₃;
d) einen Schritt der Ansäuerung von (Ea-SO₂)₂NH, NR"₃ zu (Ea-SO₂) ₂NH;
e) einen Schritt der Neutralisation von (Ea-SO₂)₂NH mit einer Alkalimetallbase zu (Ea-SO₂)₂NMe; und
f) gegebenenfalls einen Schritt der Trocknung von (Ea-SO₂) ₂NMe,
R für eine lineare oder verzweigte Alkylgruppe mit 1 bis 20 Kohlenstoffatomen steht und Me für ein Alkalimetall steht.

13. Verfahren nach Anspruch 11, **dadurch gekennzeichnet, dass** es sich um ein Verfahren zur Herstellung eines Salzes einer Fluorverbindung der Formel Ea-SO₂-OH aus einem Fluoroxysulfid-Derivat der Formel (II) handelt, das Folgendes umfasst:
a) die Herstellung eines Fluoroxysulfid-Derivats der Formel (II) gemäß dem Verfahren nach einem der Ansprüche 1 bis 10;
b') die Oxidation des Fluoroxysulfid-Derivats der Formel (II) zum Erhalt einer Fluorverbindung der Formel Ea-SO₂-OH.

14. Verfahren nach Anspruch 11, **dadurch gekennzeichnet, dass** es sich um ein Verfahren zur Herstellung einer Anhydridverbindung der Formel (Ea-SO₂)₂O aus einem Fluoroxysulfid-Derivat der Formel (II) handelt, das Folgendes umfasst:
a) die Herstellung eines Fluoroxysulfid-Derivats der Formel (II) gemäß dem Verfahren nach einem der Ansprüche 1 bis 10;
b') die Oxidation des Fluoroxysulfid-Derivats der Formel (II) zum Erhalt einer Fluorverbindung der Formel Ea-SO₂-OH;
c') die Anhydrisierung der Fluorverbindung der Formel Ea-SO₂-OH zum Erhalt einer Anhydridverbindung der Formel (Ea-SO₂)₂O.

## Claims

1. Process for the preparation of an oxysulfide and fluorinated derivative of formula Ea-SOOR (II), comprising the reaction, in the presence of an organic solvent:
i) of at least one compound of formula Ea-COOR (I), where Ea represents the fluorine atom or a group having from 1 to 10 carbon atoms chosen from fluoroalkyls, perfluoroalkyls and fluoroalkenyls, and R represents hydrogen, a monovalent cation or an alkyl group, and
ii) of a sulfur oxide,
said process being such that the initial molar ratio (sulfur oxide/compound of formula (I)) is less than 0.4 and the concentration of dissolved sulfur oxide in the reaction medium is kept constant throughout the duration of the reaction at a value of between 0.2 weight% and 3 weight% by a continuous addition of said sulfur oxide to the reaction medium.

2. Process according to Claim 1, in which said compound of formula (I) is a salt of a fluorocarboxylic acid in which the R group is a monovalent cation selected from the group consisting of alkali metal cations, quaternary ammonium cations and quaternary phosphonium cations; preferably R is an alkali metal cation.

3. Process according to Claim 1 or 2, in which the Ea group is selected from the group consisting of the fluorine atom, the CH₂F- radical, the CHF₂- radical and the CF₃- radical.

4. Process according to one of Claims 1 to 3, in which the sulfur oxide is sulfur dioxide.

5. Process according to one of Claims 1 to 4, in which the initial molar ratio (sulfur oxide/compound of formula (I)) is less than 0.2.

6. Process according to one of Claims 1 to 5, in which the concentration of dissolved sulfur oxide in the reaction medium is monitored in line or *in situ* by Raman spectrometry, by near infrared spectroscopy or by UV spectroscopy.

7. Process according to one of Claims 1 to 6, in which said organic solvent is a polar aprotic solvent preferably chosen from N,N-dimethylformamide (DMF), N,N-diethylformamide or N,N-dimethylacetamide.

8. Process according to one of Claims 1 to 7, in which the implementation is carried out continuously or semicontinuously.

9. Process according to one of Claims 1 to 8, in which the reaction is carried out at an absolute total pressure chosen between 1 and 20 bar and preferably between 1 and 3 bar.

10. Process according to one of Claims 1 to 8, in which the reaction is carried out at a pressure below atmospheric pressure.

11. Process for the preparation of a compound selected from the group consisting of a sulfonimide compound (Ea-SO₂)₂NH, its salts (Ea-SO₂)₂NMe (Me representing an alkali metal), a fluorinated compound having a sulfonic acid functional group -SO₂OH and exhibiting a formula Ea-SO₂OH, and an anhydride compound of formula (Ea-SO₂)₂O, Ea represents the fluorine atom or a group having from 1 to 10 carbon atoms chosen from fluoroalkyls, perfluoroalkyls and fluoroalkenyls, said process comprising:
- a stage of preparation of an oxysulfide and fluorinated derivative of formula (II) according to the process according to one of Claims 1 to 10,
- a stage in which said fluorinated compound of formula (II) is used as reactive compound for the synthesis of a sulfonimide compound (Ea-SO₂)₂NH and of its salts (Ea-SO₂)₂NMe (Me representing an alkali metal), of a fluorinated compound having a sulfonic acid functional group -SO₂OH and exhibiting a formula Ea-SO₂OH, or of an anhydride compound of formula (Ea-SO₂)₂O.

12. Process according to Claim 11, **characterized in that** it is a process for the preparation of a salt of a sulfonimide compound of formula (Ea-SO₂)₂NMe from an oxysulfide and fluorinated derivative of formula (II) comprising:
a) the preparation of an oxysulfide and fluorinated derivative of formula (II) according to the process according to one of Claims 1 to 10;
b) a stage of oxidation, for example by chlorination, in order to obtain the compound (Ea-SO₂)X, where X is chlorine or fluorine;
c) a stage of ammonolysis of Ea-SO₂X to give (Ea-SO₂)₂N.HNR"₃;
d) a stage of acidification of (Ea-SO₂)₂N.HNR"₃ to give (Ea-SO₂)₂NH;
e) a stage of neutralization, by an alkali metal base, of (Ea-SO₂)₂NH to give (Ea-SO₂)₂NMe; and
f) optionally a stage of drying (Ea-SO₂)₂NMe;
R" represents a linear or branched alkyl group having from 1 to 20 carbon atoms and Me represents an alkali metal.

13. Process according to Claim 11, **characterized in that** it is a process for the preparation of a fluorinated compound of formula Ea-SO₂-OH from an oxysulfide and fluorinated derivative of formula (II) comprising:
a) the preparation of an oxysulfide and fluorinated derivative of formula (II) according to the process according to one of Claims 1 to 10;
b') the oxidation of the oxysulfide and fluorinated derivative of formula (II) in order to obtain a fluorinated compound of formula Ea-SO₂-OH.

14. Process according to Claim 11, **characterized in that** it is a process for the preparation of an anhydride compound of formula (Ea-SO₂)₂O from an oxysulfide and fluorinated derivative of formula (II) comprising:
a) the preparation of an oxysulfide and fluorinated derivative of formula (II) according to the process according to one of Claims 1 to 10;
b') the oxidation of the oxysulfide and fluorinated derivative of formula (II) in order to obtain a fluorinated compound of formula Ea-SO₂-OH;
c') the anhydrization of the fluorinated compound of formula Ea-SO₂-OH in order to obtain an anhydride compound of formula (Ea-SO₂)₂O.
